# EUROPEAN PATENT APPLICATION

(11) **EP 2 136 200 A1**
(43) Date of publication of application: **23.12.2009**
(21) Application number: 08167677.7
(22) Date of filing: 27.10.2008
(51) Int. Cl.: G01N 22/04, G01N 33/38

(54) **Surface probe for moisture content measurement in building partitions by time domain reflectometry comprising a transmission line made from elastic conductive foam to conform to the rough surface of the building partition**

(30) Priority: 16.06.2008 PL 38544408
(71) Applicant: Politechnika Lubelska, 20-219 Lublin (PL)
(72) Inventor: Sobczuk, Henryk, 21-400, Swidnik (PL); Suchorab, Zbigniew, 21-010, Leczna (PL)
(74) Representative: Kaminski, Piotr

(57) **Abstract**

Invention relates to a probe for moisture content measurement in building partitions, specially with rough surface and is characterized in that the probe contain an elastic transmission line (2) which allows for probe adjusting to the rough surface of the measured building partition. The transmission line is made from elastic conductive foam.

## Description

The subject of the invention is a probe for moisture content measurement in building partitions, especially with rough surfaces. The probe works with the application of time domain reflectometry method and is utilized for moisture content measurement of building partitions and materials. It is especially useful in case of partitions with rough and irregular surfaces.

Known probe constructions are invasive in measurement because the principal measurement elements are metal rods of significant length of small diameter that during measurement have to be inserted through the holes into the material. This is the cause of montage problems especially in cases of hard materials measurement. In many cases this makes the measurement impossible or causes measurement errors.

Surface probe, according to Polish patent PL 350713 allows for a non invasive measurement of moisture content of building partitions. It is characterized by rigid construction. The measuring elements are the metal angle bars which are placed on the flat surface of partition for the measurement. There is no need to insert the measuring elements into the structure of the partition. The described probe is a useful solution for humidity measurement in flat structure elements.

The probe according to present invention allows moisture content measurement, especially in building partitions with rough surfaces and has elastic measurement elements allowing to fit the probe to the rough shape of the surface of the measured building partition while measurement elements are made from elastic conductive foam.

The advantage of the present invention is utilization of potential of time domain reflectometry method and its application for measurement of objects so far inaccessible. The probe for moisture content measurement, especially in building partitions with rough surface, can be applied for measurements of moisture content of hard building elements characterized by irregular, rough front (due to the character of plaster layer or surface deterioration). Measurements performed with use of probe according to this invention are possible irrespectively of the type and shape of measured detail.

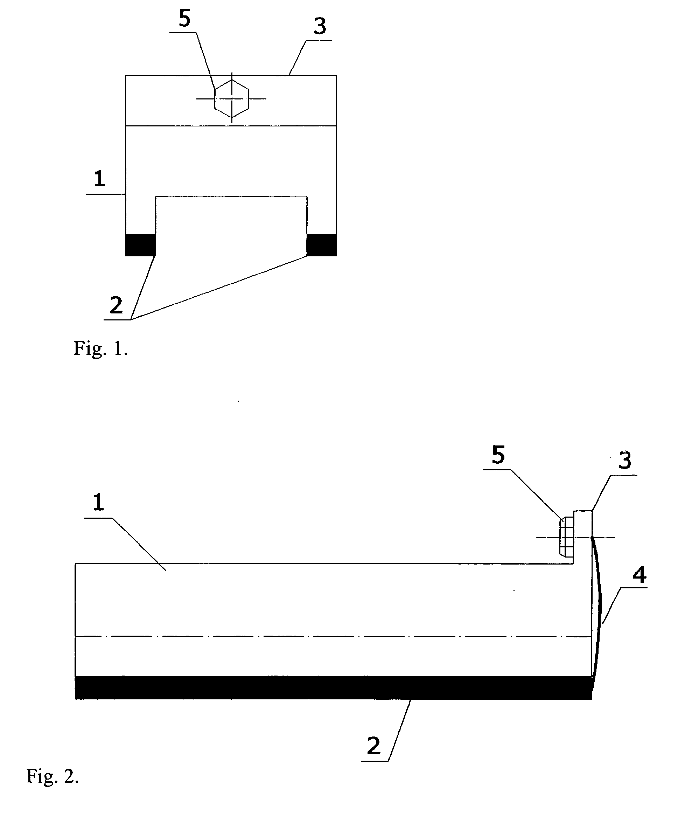

The embodiment of the invention is presented in the drawing, where Fig. 1 presents the probe in transversal cross section view while Fig.2 illustrates the probe in longitudinal cross section view.

The probe consists of two flat, elastic, placed in parallel measurement elements 2. Measurement elements 2 are separated with the elastic insulating frame 1. Each measurement element 2 is connected with insulating frame 2 in the upper surface, while the lower surface remains free. The measurement elements 2 are connected by individual conductors 4 with connector 5 of the concentric cable connecting the probe with the readout device. In cross-section, the measuring elements 2 have preferably rectangular shape and are made from elastic conducting foam. Insulating frame 1 keeps parallel measurement elements 2 in fixed distance. For construction stability the side plate 3 is provided. Along the plate, individual conductors 4 are guided from the measurement elements 2 to concentric cable connector 5.

For measurement, the probe for measurement of moisture content in building partitions, especially elements with rough surfaces, is placed at the rough surface and pushed against so that the measurement elements 2 deform in order to conform to the measured rough building partition. In this position, the measurement with the time domain reflectometry method is performed giving readout of the moisture content.

## Claims

1. Probe for moisture content measurement in building partitions, specially with rough surfaces, **characterized in that** it contains elastic measurement elements (2) which allow for probe adjusting to the rough surface of the measured building partition.

2. The probe according to claim 1 **characterized in that** measurement elements 2 are made from elastic conductive foam.
